# DEMANDE DE BREVET EUROPEEN

(11) **EP 0 681 813 A1**
(43) Date de publication de la demande: **15.11.1995**
(21) Numéro de dépôt: 95400843.9
(22) Date de dépôt: 13.04.1995
(51) Int. Cl.: A61B 17/322

(54) **Instrument de coupe de greffons capillaires calibrés**

(30) Priorité: 13.05.1994 FR 9405884
(71) Demandeur: MEDICAMAT S.A., F-92240 Malakoff (FR)
(72) Inventeur: Boudjema, Pascal, F-75016 Paris (FR)
(74) Mandataire: Plaçais, Jean-Yves

(57) **Abrégé**

L'invention concerne un dispositif de découpe chirurgicale de bandelettes de cuir chevelu en de multiples greffons capillaires calibrés, de petite taille identique, combinant
- d'une part, un porte-lames (4) agencé de façon à autoriser la mise en place verticale de lames multiples (1) parallèles équidistantes et de façon à ce que les bords tranchants (2) des-dites lames soient alignés dans un même plan horizontal, ces lames étant alors saillantes vis à vis d'une surface support (8) et,
- d'autre part, une plaque de matière souple (10) destinée à venir à l'aplomb des bords coupants des lames (1) et associée à un applicateur (9) associé à des moyens de retenue ou de butée et, le cas échéant, de guidage, permettant l'application ou le pressage de cette plaque (10) sur les bords tranchants des lames, parallèlement à ladite surface support (8), tout en assurant le non-dépassement par cette plaque d'une position limite, à une distance de la surface de support, supérieure à l'épaisseur des bandelettes auxquelles le dispositif de découpe chirurgicale est destiné.

## Description

La présente invention, concerne un dispositif de découpe chirurgicale de bandelettes de cuir chevelu en de multiples greffons capillaires, de petite taille, identiques, calibrés, comprenant un instrument de coupe à lames multiples parallèles équidistantes.

Elle trouve une application particulièrement importante bien que non exclusive dans le domaine du traitement chirurgical de la calvitie par greffe des cheveux naturels, et plus particulièrement dans le domaine de la micro-greffe.

Le traitement chirurgical de la calvitie par transplantation capillaire, dite technique des greffons, consiste à transplanter chez un même individu, une partie des racines de ses cheveux situées dans la couronne (toujours chevelue) vers les zones chauves.

L'obtention de micro-greffons du cuir chevelu est par exemple, effectuée à partir d'une bandelette de peau rectangulaire, allongée, d'environ 14 cm de long sur 1,5 mm de large, et 5 mm de profondeur, contenant des racines de cheveux, préalablement prélevée, au niveau de la couronne occipitale, à l'aide d'un couteau constitué d'un manche à l'extrémité duquel sont montées quatre à six lames coupantes parallèles équidistantes, et espacées entre elles d'environ 1,5 mm. Ce couteau permet ainsi d'obtenir 3 à 5 bandelettes d'environ 1,5 mm de largeur et 14 cm de longueur (premier temps opératoire).

Chaque bandelette prélevée est alors disposée à plat, sur un plan de travail dur, maintenue par une pince, puis redécoupée perpendiculairement à son grand axe, parallèlement à l'axe des racines, à l'aide d'un couteau à une lame, en plusieurs centaines de micro-greffons de forme quadrangulaire dont la taille varie de 1 à 3 mm de largeur et contenant chacun une à quatre racines (deuxième temps opératoire).

Les greffons ainsi obtenus sont alors réimplantés un à un dans des petits orifices receveurs aménagés au niveau des zones chauves (troisième temps opératoire).

Malheureusement, les micro-greffons obtenus n'ayant pas la même dimension et n'étant pas calibrés et homogènes, étant donné qu'ils sont découpés à vue et à main levée, ne s'adaptent pas parfaitement dans les orifices receveurs et en outre, la découpe manuelle des bandelettes est longue et fastidieuse.

La présente invention vise à éliminer les inconvénients énumérés ci-dessus en proposant un dispositif et un procédé répondant mieux que ceux antérieurement connus aux exigences de la pratique, notamment en permettant de découper rapidement et simultanément les bandelettes de cuir chevelu en de multiples micro-greffons parfaitement identiques, calibrés et homogènes, pour un coût faible, de façon simple et aisée à mettre en oeuvre, et ce essentiellement sans nuire à leur intégrité, et, par voie de conséquence, à leur capacité à être ensuite réimplantés dans les zones chauves.

Dans ce but, l'invention propose essentiellement un dispositif de découpe chirurgicale de bandelettes de cuir chevelu en de multiples greffons capillaires calibrés, de petite taille identique, combinant
- d'une part, un porte-lames agencé de façon à autoriser la mise en place verticale de lames multiples parallèles équidistantes et de façon à ce que les bords tranchants des-dites lames soient alignés dans un même plan horizontal, ces lames étant alors saillantes vis à vis d'une surface support et,
- d'autre part, une plaque de matière souple destinée à venir à l'aplomb des bords coupants des lames et associée à un applicateur associé à des moyens de retenue ou de butée et, le cas échéant, de guidage, permettant l'application ou le pressage de cette plaque sur les bords tranchants des lames, parallèlement à ladite surface support, tout en assurant le non-dépassement par cette plaque d'une position limite, à une distance de la surface de support, supérieure à l'épaisseur des bandelettes auxquelles le dispositif de découpe chirurgicale est destiné.

Dans ces conditions, l'on peut procéder à la découpe des bandelettes, une fois celles-ci posées sur les tranchants des lames, par applicaticn et pressage de la plaque sur la bandelette et jusqu'à ce que la plaque atteigne la susdite position limite, les lames entamant alors la matière souple de la plaque. On peut naturellement envisager les étapes inverses: pose des bandelettes sur la plaque et mouvement vers celles-ci des lames.

Normalement le porte-lames est ainsi organisé pour que les lames puissent être serrées les unes contre les autres, ou, au contraire, desserrées, notamment selon le calibre voulu des micro-greffons.

Avantageusement, ces opérations sont réalisées par l'intermédiaire d'entretoises entre lesquelles viennent s'intercaler les lames, de préférence amovibles, et dont les tranches orientées vers l'extérieur définissent la surface support vis-à-vis de laquelle les lames font saillie. Avantageusement, ces entretoises coagissent elles-mêmes avec des surfaces ou éléments de guidage leur permettant des déplacements relatifs dans une direction perpendiculaire aux plans de ces entretoises, par exemple les surfaces intérieures d'un boîtier dans lequel elles sont normalement logées avec les lames, lorsque celles-ci sont en place. Le cas échéant, ces dernières surfaces ou éléments de guidage contribuent également au maintien en place des lames, celles-ci pouvant alors être retirées du dispositif, par exemple par escamotage de certains de ces éléments de guidage.
Avantageusement, et notamment lorsque les entretoises et les lames sont guidées entre les faces internes opposées d'un boîtier, l'applicateur est associé au couvercle du boîtier, par exemple fait saillie vis-à-vis de la surface interne de ce couvercle, afin que la plaque en matière souple vienne s'appliquer sur les tranchants des lames, lorsque les bords du couvercle entourant l'applicateur viennent s'appuyer, en position de fermeture, sur les rebords correspondant du boîtier.

Il va de soi que la plaque de matière souple doit aussi être amovible, afin qu'il puisse être pourvu à son remplacement, au cours des usages successifs du dispositif.

De nombreux avantages et caractéristiques de la présente invention ressortiront au cours de la description qui va suivre, faite en regard des dessins annexés donnant à titre explicatif mais nullement limitatif une forme de réalisation conforme à l'invention.

Sur ces dessins:
- la figure 1 est une vue de dessus du dispositif selon un mode de réalisation de l'invention, plaque de compression relevée,
- la figure 2 est une vue de devant du dispositif de la figure 1,
- la figure 3 est une vue de devant du dispositif, plaque de compression abaissée,
- la figure 4 est une vue en coupe de la figure 3,
- la figure 5 est une vue de côté du dispositif de la figure 2,
- la figure 6 est une vue de côté du dispositif de la figure 3,

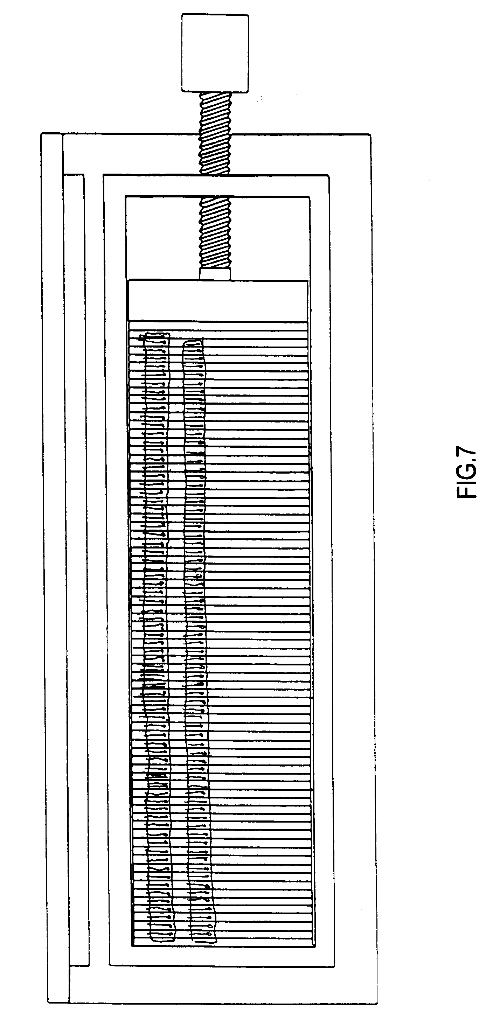

- la figure 7 est une vue de dessus du dispositif, indiquant la position de deux bandelettes de cuir chevelu après découpe.

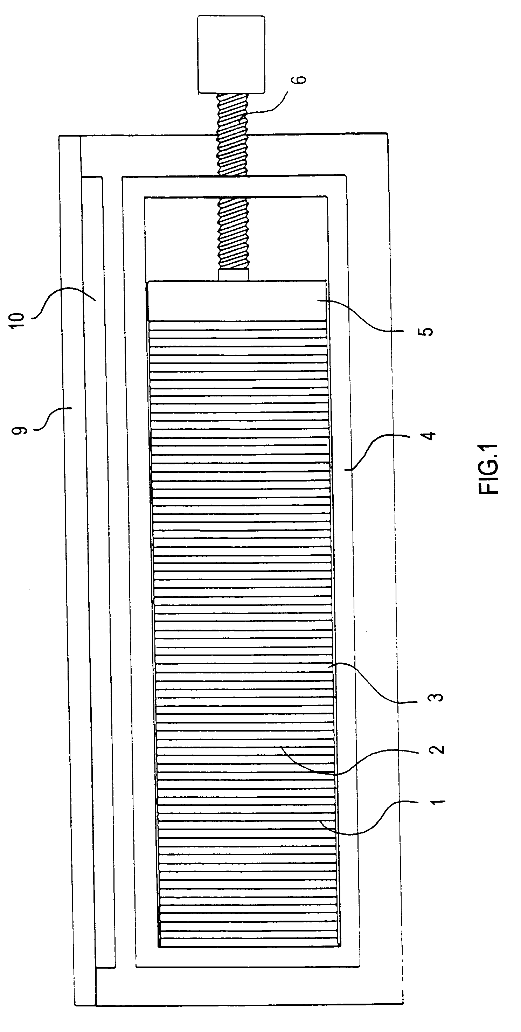

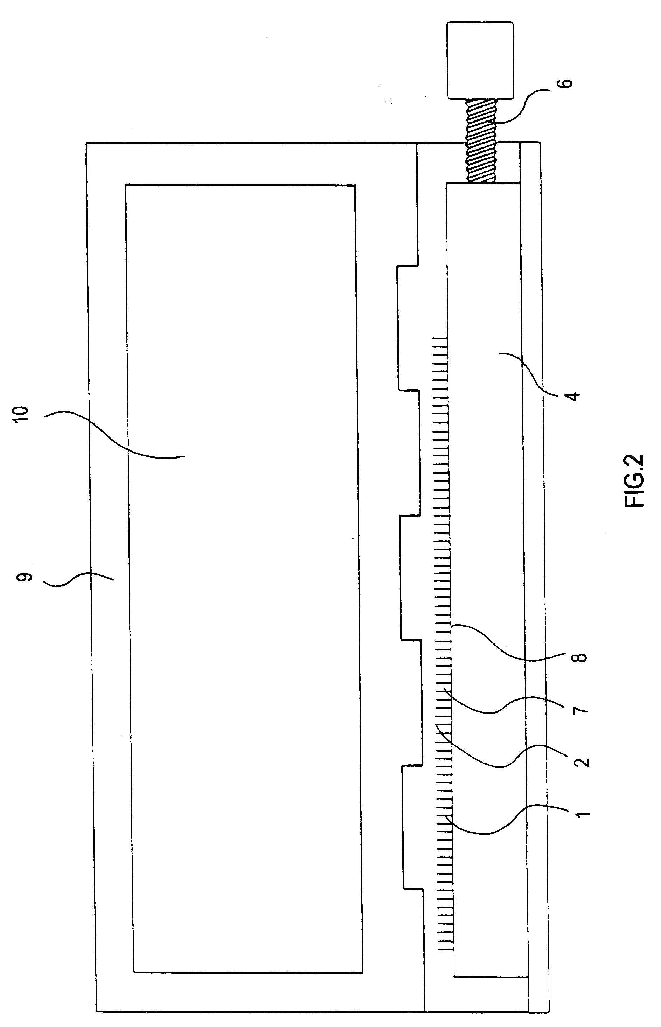

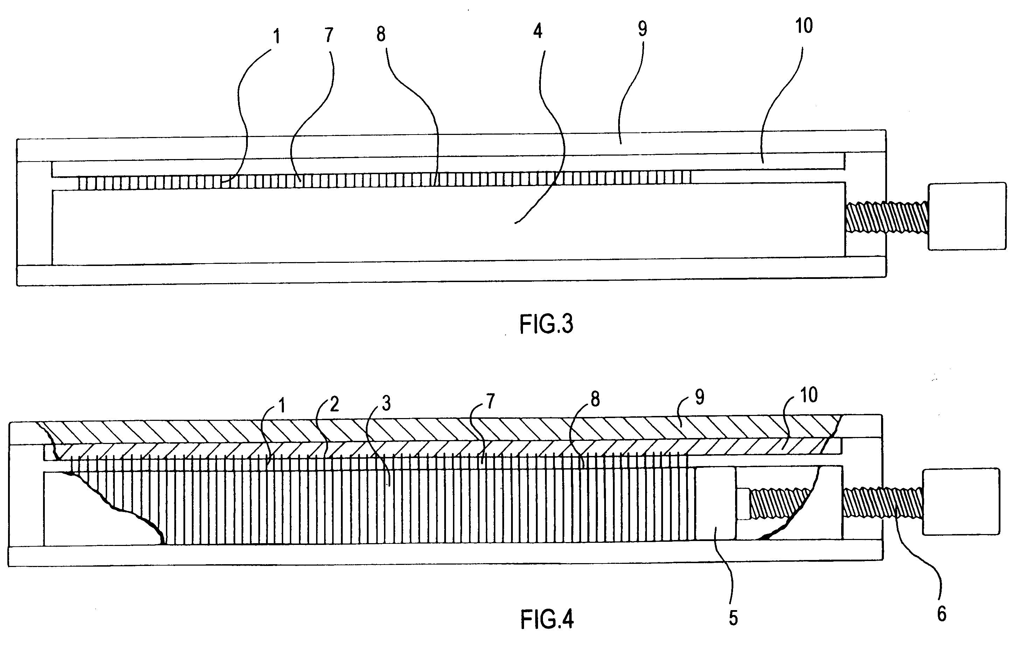

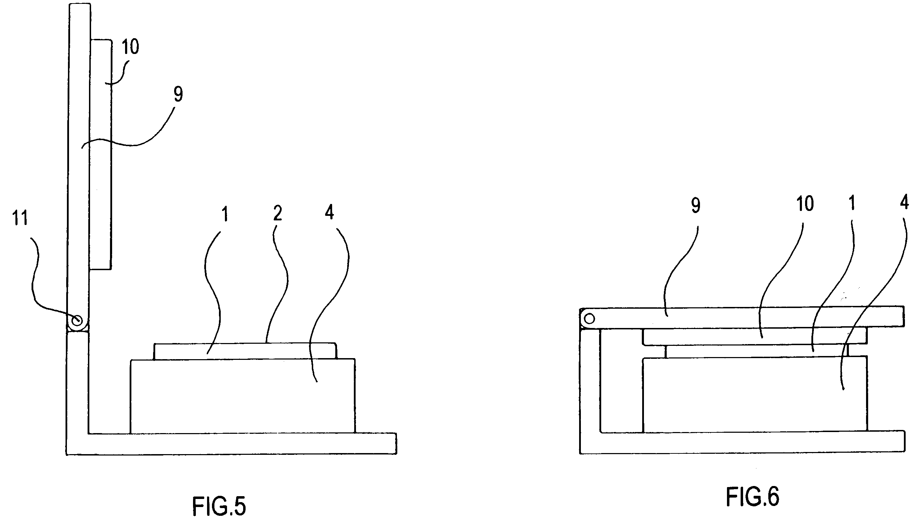

Sur les figures 1 à 6, on voit que l'instrument de coupe chirurgical selon l'invention est constitué de multiples lames (1) coupantes, de forme rectangulaire alignées verticalement, du type de lames de rasoir, de 0,1 mm d'épaisseur, parallèles entre elles, à bords tranchants (2) rectilignes d'environ 44 mm de long, disposés dans un même plan horizontal. Lesdites lames sont équidistantes séparées les unes des autres par des plaques intercalaires (3) faisant office d'entretoises, de forme également rectangulaire, de même longueur que les lames, et d'épaisseur déterminée, par exemple 1,4 mm. Les lames et plaques intercalaires sont disposées au sein d'un boîtier allongé (4) et maintenues serrées solidairement entre elles, par une cale (5) mobilisée par une tige filetée (6) de serrage, faisant office d'étau transversal.
Les lames (1) ont une hauteur supérieure de quelques millimètres à celle des plaques intercalaires (3) dont les tranches supérieures définissent alors la surface support (8) de telle sorte qu'un espace libre (7) puisse être aménagé entre lesdites lames.
Une plaque rigide (9), mobile, faisant office de presse, de forme rectangulaire, permet, en se rabattant sur le boîtier (4), par l'intermédiaire d'une charnière (11), de comprimer une plaque intermédiaire (10) à type de tampon en matière plastique souple telle que silicone, contre le bord tranchant (2) des lames coupantes (1).

Concernant l'instrument de coupe selon l'invention, la longueur du boîtier (4) peut avoisiner 21 cm de telle sorte qu'il puisse contenir plus d'une centaine de lames coupantes (1) espacées les unes des autres par une centaine de plaques intercalaires (3) de 1 à 1,5 mm d'épaisseur.

Les plaques intercalaires (3) peuvent être réalisées en acier inoxydable.
La plaque rigide (9) peut être réalisée en acier inoxydable.

Concernant la plaque rigide (9) supportant la plaque intermédiaire souple (10), bien qu'elle ait été représentée mobile autour d'une charnière (11), et rabattue manuellement sur le boîtier (4), il est également possible de la concevoir séparée du dispositif proprement-dit puis positionnée par le biais de la plaque intermédiaire souple (10) contre les bords tranchants (2) des lames, par vissage vertical de boulons.
Le dispositif selon l'invention fonctionne comme suit:
Les bandelettes de cuir chevelu, préalablement prélevées au niveau de la couronne, avoisinant chacune 140 mm de longueur, 5 mm de largeur et 1,4 mm d'épaisseur, sont déposées sur toute leur longueur, délicatement à plat , sur le bord tranchant (2) des lames coupantes (1) au nombre de cent. L'orientation des bandelettes est telle que l'axe des racines soit parallèle aux lames coupantes (1).
La compression de la plaque intermédiaire en matière souple (10) contre les bandelettes, par mobilisation de la plaque rigide (9), permet alors la découpe instantanée et simultanée des bandelettes en plusieurs centaines de greffons retrouvés entre les lames, dans les espaces libres (7). La plaque intermédiaire souple (10) étant relevée, le desserrage de la tige fileté (6) faisant office d'étau transversal, permet de décoincer les greffons qui sont alors récupérés pour être réimplantés.

## Revendications

1. Dispositif de découpe chirurgicale de bandelettes de cuir chevelu en de multiples greffons capillaires calibrés, de petite taille identique, combinant
- d'une part, un porte-lames (4) agencé de façon à autoriser la mise en place verticale de lames multiples (1) parallèles équidistantes et de façon à ce que les bords tranchants (2) des-dites lames soient alignés dans un même plan horizontal, ces lames étant alors saillantes vis à vis d'une surface support (8) et,
- d'autre part, une plaque de matière souple (10) destinée à venir à l'aplomb des bords coupants des lames (1) et associée à un applicateur (9) associé à des moyens de retenue ou de butée et, le cas échéant, de guidage, permettant l'application ou le pressage de cette plaque (10) sur les bords tranchants des lames, parallèlement à ladite surface support (8), tout en assurant le non-dépassement par cette plaque d'une position limite, à une distance de la surface de support, supérieure à l'épaisseur des bandelettes auxquelles le dispositif de découpe chirurgicale est destiné.

2. Dispositif selon la revendication 1, caractérisé en ce que le porte-lames (4) comporte des entretoises (3) entre lesquelles viennent s'intercaler les lames (1), elles-mêmes amovibles, les tranches orientées vers l'extérieur de ces entretoises définissant la susdite surface support (8) vis-à-vis de laquelle les lames doivent faire saillie.

3. Dispositif selon la revendication 2, caractérisé en ce que ces entretoises (3) coagissent elles-mêmes avec des surfaces ou éléments de guidage leur permettant des déplacements relatifs dans une direction perpendiculaire aux plans de ces entretoises.

4. Dispositif selon la revendication 2 ou 3, caractérisé en ce que les surfaces de guidage sont formées par les surfaces internes d'un boîtier dans lequel sont normalement logées les entretoises et les lames, lorsque celles-ci sont en place.

5. Dispositif selon l'une quelconque des revendications 1 à 4, caractérisé, notamment lorsque les entretoises et les lames sont guidées entre les faces internes opposées d'un boîtier, en ce que l'applicateur (9) est associé au couvercle du boîtier.

6. Dispositif selon la revendication 5, caractérisé en ce que l'applicateur (9) fait saillie vis-à-vis de la surface interne du couvercle, afin que la plaque en matière souple (10) vienne s'appliquer sur les bords tranchants (2) des lames, lorsque les bords du couvercle entourant l'applicateur viennent s'appuyer, en position de fermeture, sur les rebords correspondant du boîtier.

7. Dispositif selon l'une quelconque des revendications 1 à 6, caractérisé en ce que la plaque en matière souple (10) est amovible.
